# EUROPEAN PATENT SPECIFICATION

(11) **EP 0 923 548 B1**
(45) Date of publication and mention of the grant of the patent: **12.03.2003**
(21) Application number: 97938629.9
(22) Date of filing: 26.08.1997
(51) Int. Cl.: C07D 209/08, A61K 31/40, C07D 209/30, C07D 409/12, C07D 209/12

(54) **4-AMINOETHOXY INDOLES AS DOPAMIN D2 AGONISTS AND AS 5HT 1A LIGANDS**
4-AMINOETHOXY-INDOLDERIVATE ALS DOPAMIN D2 AGONISTEN UND ALS 5HT1A LIGANDEN
4-AMINOETHOXY INDOLES UTILISES EN TANT QU'AGONISTES DU RECEPTEUR D2 DE LA DOPAMINE ET EN TANT QUE LIGANDS DES RECEPTEURS 5HT 1A

(30) Priority: 27.08.1996 US 703562
(43) Date of publication of application: 23.06.1999
(73) Proprietor: Wyeth, Madison, New Jersey 07940-0874 (US)
(72) Inventor: MEWSHAW, Richard Eric, Princeton, NJ 08540 (US); WEBB, Michael Byron, Levittown, PA 19054 (US)
(74) Representative: Mannion, Sally Kim, Dr.
(86) International application number: US9715026
(87) International publication number: WO98008817

(56) References cited:
- US-A- 3 906 000
- US-A- 5 013 761
- US-A- 5 541 199

## Description

### BACKGROUND OF INVENTION

Efforts to induce antipsychotic activity with dopamine autoreceptor agonists have been successful [Dorsini et al., Adv. Biochem. Psychopharmacol 16, 645-648, (1977); Tamminga et al., Science 200, 567-568; and Tamminga et al., Psychiatry 398-402, (1986)]. A method for determining intrinsic activity at the dopamine D2 receptor was recently reported [Lahti et al., Mol. Pharm. 42, 432-438, (1993)]. Intrinsic activity is predicted using the ratio of the "low-affinity agonist" (LowAg) state of the receptor and the "high-affinity agonist" (HighAg) state of the receptor, i.e. LowAg/HighAg. These ratios correlate with the agonist, partial agonist and antagonist activities of a given compound, which activities characterize a compound's ability to elicit an antipsychotic effect.

U.S. Pat. Nos. 3,906,000 and 3,904,645 describe a series of indoles which are useful as oral hypoglycemic agents. Troxler et al. 66-25558F: WPIDS describes a series of indoles including 4-(2-hydroxy-3-isopropyl- or secondary butyl-amino-propoxy)-indoles which are useful as β-adrenergic blocking agents for the treatment of heart diseases.

### DESCRIPTION OF THE INVENTION

In accordance with this invention, there is provided a group of aminoethoxy indole derivatives which are useful antipsychotic agents. In addition, this invention provides processes for preparation of the compounds and methods for their use in treating diseases of the central nervous system. The aminoethoxy indoles of ths invention are illustrated by the following Formula I: 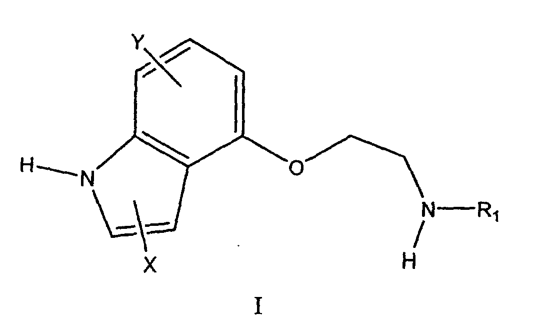 in which:
R₁ is hydrogen, alkyl of 1 to 10 carbon atoms, cycloalkylalkyl of 6 to 12 carbon atoms, arylalkyl of 7 to 12 carbon atoms, (haloaryl)alkyl of 7 to 12 carbon atoms, (alkoxyaryl)alkyl of 8 to 12 carbon atoms, thienylmethyl, furanylmethyl, pyridinylmethyl, alkylphenyl of 7 to 12 carbon atoms, 4-fluorobutyrophenone or 6-fluoro-1,2-benzisoxazol-yl-propyl;
X is hydrogen, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms;
or a pharmaceutically acceptable salt thereof; for use in therapy.

More specifically, the present invention provides 4-aminoethoxy-indole compounds of Formula 1 : 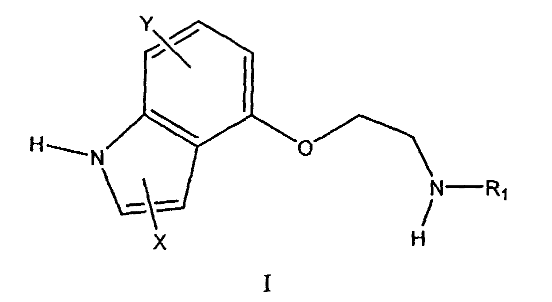 in which:
R₁ is hydrogen, alkyl of 1 to 10 carbon atoms, cycloalkylalkyl of 6 to 12 carbon atoms, arylalkyl of 7 to 12 carbon atoms, (haloaryl)alkyl of 7 to 12 carbon atoms, (alkoxyaryl)alkyl of 8 to 12 carbon atoms, thienylmethyl, furanylmethyl, pyridinylmethyl, alkylphenyl of 7 to 12 carbon atoms, 4-fluorobutyrophenone or 6-fluoro-1,2-benzisoxazol-yl-propyl;
X is hydrogen, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms; or a pharmaceutically acceptable salt thereof; provided that when R₁ and X are both hydrogen, then Y is not hydrogen, 5-methoxy or 7-methoxy.

Preferred compounds of Formula I are those in which R₁ is hydrogen, alkyl of 1 to 10 carbon atoms, cyclohexylmethyl, arylalkyl of 7 to 12 carbon atoms, (haloaryl)alkyl of 7 to 12 carbon atoms, (alkoxyaryl)alkyl of 8 to 12 carbon atoms; X is hydrogen, halogen, cyano, alkyl of 1 to 6 carbon atoms, acetyl, trifluoroacetyl, trifluoromethyl or formyl; and Y is hydrogen, halogen, alkoxy of 1 to 6 carbon atoms or alkyl of 1 to 6 carbon atoms; or a pharmaceutically acceptable salt thereof.

More preferred compounds are those of Formula I in which R₁ is alkyl of 1 to 6 carbon atoms, benzyl, halobenzyl, alkoxybenzyl of 8 to 12 carbon atoms or alkylbenzyl of 8 to 12 carbon atoms; X is hydrogen, halogen or trifluoroacetyl and Y is hydrogen or halogen; or pharmaceutically acceptable salt thereof.

The pharmaceutically acceptable acids from which addition salts are conventionally produced having the same utility as the free base, include both inorganic or organic acids. For example : fumaric, maleic, benzoic, ascorbic, pamoic, succinic, bismethylenesalicylic, methanesulfonic, ethanedisulfonic, acetic, axalic, propionic, tartaric, salicylic, citric, gluconic, lactic, malic, mandelic, cinnamic, citraconic, aspartic, steric, plamitic, itaconic, glycolic, p-aminobenzoic, glutamic, benzene-sulfonic, hydrochloric, hydrobromic, sulfuric, cyclohexylsulfamic, phosphoric, nitric acid, and the like, are suitable for this purpose.

The compounds of formula I are generally prepared by the overall reaction sequence indicated in Schemes I, II, and III as follows :

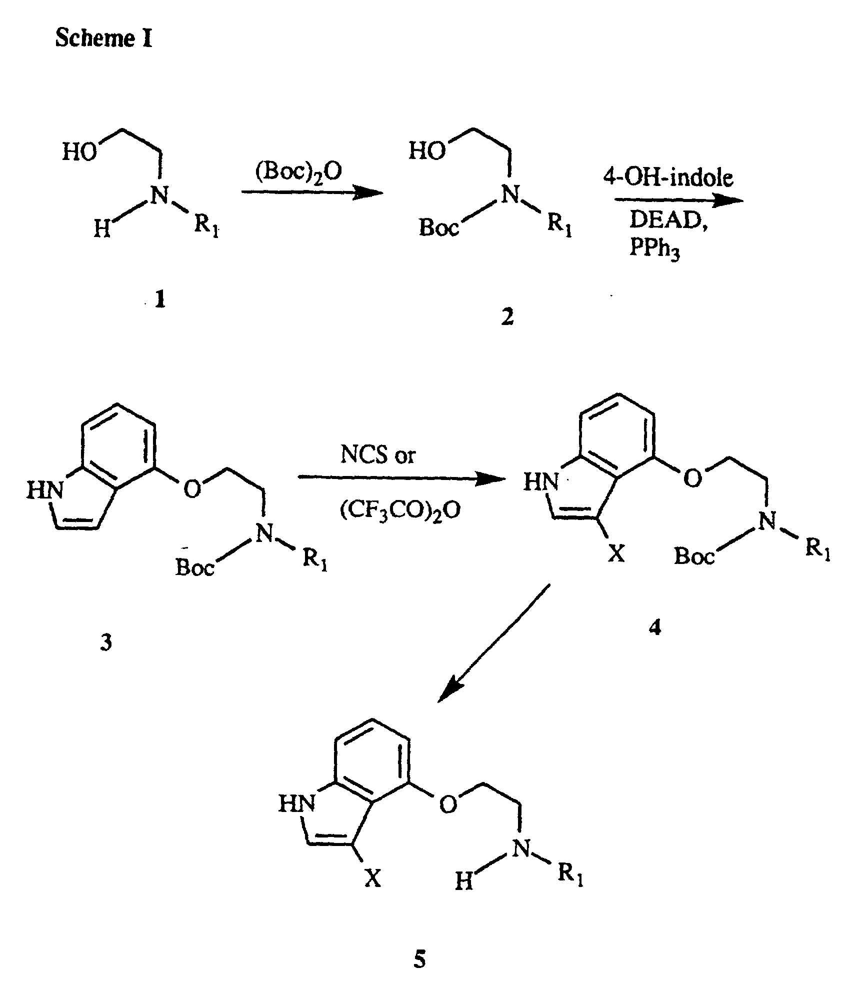

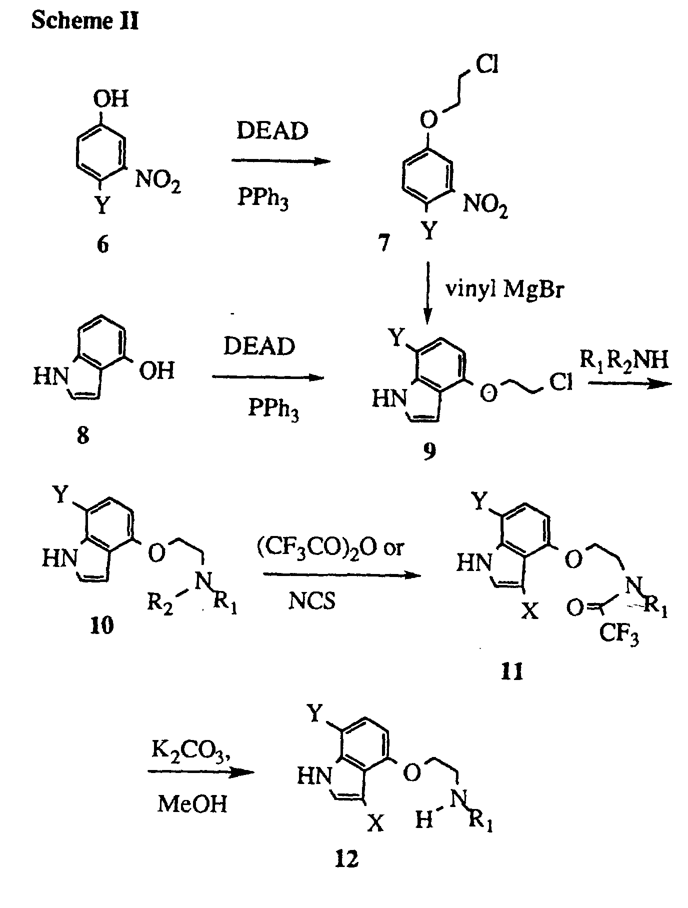

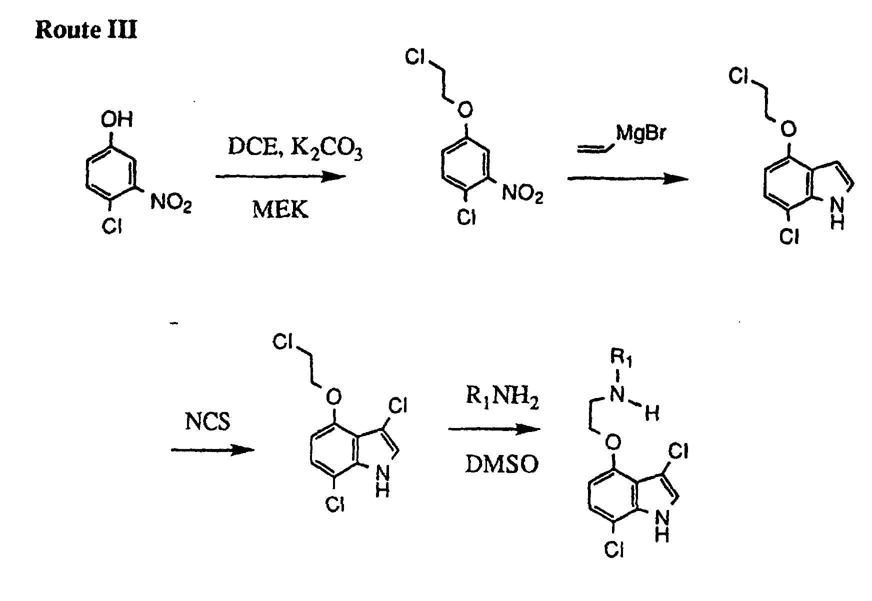

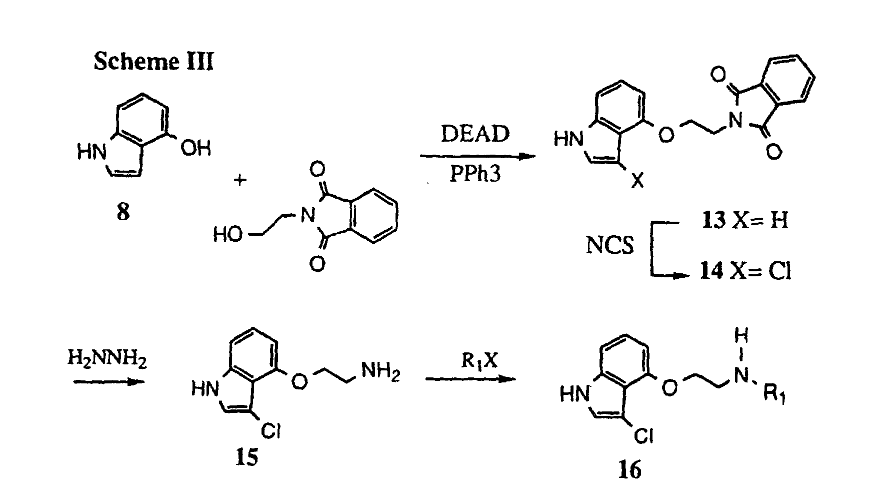

The compounds of this invention are dopamine agonists with various degrees of intrinsic activity. Some are selective autoreceptor agonists and others bind to the postsynaptic D₂ receptors. The autoreceptor agonists act as partial agonists (i.e. activate only autoreceptors versus postsynaptic D₂ dopamine receptors). As such, they provide functional modulation of the dopamine systems of the brain without the excessive blockade of the postsynaptic dopamine receptors which have been observed to be responsible for the serious side effects frequently exhibited by agents found otherwise clinically effective for the treatment of schizophrenia. Activation of the dopamine autoreceptors results in reduced neuronal firing as well as inhibition of dopamine synthesis and release and therefore provide a means of controlling hyperactivity of the dopaminergic systems with essentially no extrapyramidal side effects (EPS).

The compounds of this invention were also found to have affinity for the 5-HT_{1A} receptors and therefore have the ability to modulate serotonergic activity. As such, they are useful in the treatment of diseases characterized by disturbances in the dopaminergic and serotinergic systems, such as schizophrenia, Parkinson's disease, Tourette's Syndrome, alcohol addiction, cocaine addiction, anxiety, stress, depression, sexual dysfunctions and sleep disturbances.

The following examples illustrate methods for production of the compounds of this invention.

### Intermediate 1

### N-Benzyl-N-(2-hydroxy-ethyl)-carbamic acid tert-butyl ester

A solution of N-benzylaminoethanol (4.8 g, 31.9 mmol) and di-tertbutyl-dicarbonate (7.5 g, 34.4 mmol) in anhydrous tetrahydrofuran ( 30 mL) was stirred at ambient temperature for 18 hours. The solvent was removed and the product purified by flash chromatography (ethyl acetate-hexane, 1:1) to afford 8.0 g (99%) of a thick oil.

| Elemental analysis for C₁₄H₂₁NO₃ | | | |
|---|---|---|---|
| Calc'd | C, 66.91; | H, 8.42; | N, 5.57 |
| Found | C, 66.64; | H, 8.59; | N, 5.60 |

This general procedure utilizing N-methylaminoethanol afforded:
(**1b**) N-Methyl-N-(2-hydroxy-ethyl)-carbamic acid tert-butyl ester as a clear oil (83.7%); MS m/z 175 (M+).

| Elemental analysis for C₈H₁₇NO₃ | | | |
|---|---|---|---|
| Calc'd | C, 54.84; | H, 9.78; | N, 7.99 |
| Found | C, 54.35; | H, 10.00; | N, 7.84 |

### Intermediate 2

### Method A

### N-Benzyl-N-[2-(1H-indol-4-yloxy)-ethyl]-carbamic acid tert-butyl ester

To a solution of benzyl-(2-hydroxy-ethyl)-carbamic acid tert-butyl ester (12,68 g, 50.5 mmol), 4-hydroxyindole (4.48 g, 33.6 mmol) and triphenylphosphine (14.1 g, 53.8 mmol) in anhydrous tetrahydrofuran (130 mL) was slowly added a solution of diethylazidocarboxylate (9.38 g, 53.8 mmol) in tetrahydrofuran (15 mL) at room temperature. The reaction mixture was stirred for 16 hours and then the solvent was removed and the crude product dissolved in diethyl ether and diluted with hexanes. After standing for 30 minutes, the solid was filtered and the filtrate concentrated. The product was purified by flash chromatography to afford 8.6 g of a yellow oil (69.7 %).

| Elemental analysis for C₂₂H₂₆N₂O₃ | | | |
|---|---|---|---|
| Calc'd | C, 72.11; | H, 7.15; | N, 7.64 |
| Found | C, 71.39; | H, 7.28; | N, 7.21 |

This general procedure utilizing N-methyl-N-(2-hydroxy-ethyl)-carbamic acid ten-butyl ester, N-(2-hydroxyethyl)-phthalimide afforded, chloroethanol or 4-chloro-3-nitrophenol afforded, respectively:
(**2b**) N-Methyl-N-[2-(1H-indol-4-yloxy)-ethyl]-carbamic acid tert-butyl ester as a yellow oil; (77.2 %); MS EI m/z 290 (M+).
(**2c**) N-[2-(1H-Indol-4-yloxy)-ethyl]-phthalimide as a white solid: (13.2 %); mp 155-157°C; IR (KBr) 3400, 1725 cm-1; MS EI m/e 306 (M+).

| Elemental analysis for C₁₈H₁₄N₂O₃ | | | |
|---|---|---|---|
| Calc'd | C, 70.58; | H, 4.61; | N, 9.15. |
| Found | C, 70.33; | H, 4.43; | N, 9.11 |

(**2d**) 2-(1H-Indol-4-yloxy)-chloroethane: (57 %), mp 62-63 °C.
(**2e**) 1-(2-Chloroethoxy)-4-chloro-3-nitrobenzene: (93%); mp 46-48 °C; MS EI *m*/*e* 235, 237, 239 (M+);¹H NMR (400 MHz, DMSO-d₆) δ 3.95 (t, 2H, J=5.2 Hz), 4.36 (t, 2H, J=5.2 Hz), 7.32 (dd, 1H, J=3.2, J=8.9 Hz), 7.66, (d, 1H, J=9 Hz), 7.69, (d, 1H, J=3.2 Hz).

| Elemental analysis for C₈H₇Cl₂NO₃ | | | |
|---|---|---|---|
| Calc'd | C, 40.71; | H, 2.99; | N, 5.93. |
| Found | C, 40.43, | H, 2.71; | N, 5.62. |

**(2f) 1-(2-Chloroethoxy)-4-chloro-3-nitrobenzene**

### Method B

To a 2L 3-neck round-bottom flask was added 4-chloro-3-nitro-phenol (50 g, 0.29 mol), potassium carbonate (100g, 0.72 mol), dichloroethane (315 g, 3.2 mol), potassium iodide (5 g) and 2-butanone (1 L). The mixture was mechanically stirred and heated to reflux for 44 hours then allowed to cool to room temperature and the solids were filtered. The solvent was evaporated under vacuum and the oil dissolved in diethyl ether (300 mL) and washed with 10 % sodium hydroxide. The organic layer was dried over anhydrous magnesium sulfate, filtered, and the solvent removed under vacuum. The product was dissolved in 1:1 methylene chloride-hexanes and filtered through silica. Upon concentration 54.5 g (78.% %) of product was afforded as a white solid: mp 44.5-46 °C.

| Elemental analysis for C₈H₇Cl₂NO₃ | | | |
|---|---|---|---|
| Calc'd | C, 40.71; | H, 2.99; | N, 5.93. |
| Found | C, 40.89, | H, 2.70; | N, 5.83. |

### Intermediate 3

### 7-Chloro-4-(2-chloroethoxy)-1H-indole

To a solution of 1-(2-chloroethoxy)-4-chloro-3-nitrobenzene (10.00 g, 0.04236 mol) in THF (230 mL) stirred in a cold bath at -50 to -40 °C was added a THF solution of vinylmagnesium bromide (132 mL, 1.0 M, 0.132 mol) over 2 minutes. After stirring in the cold bath for 2-2.5 hours, saturated NH₄Cl (150 mL) was added to the cold solution and it was removed from the cold bath. Enough 1 M HCl was added to dissolve the precipitated solids. This two phase system was stirred for 0.5 hour at most. The layers were separated and the aqueous phase was extracted once with Et₂O. Combination of the Et₂O and THF followed by drying over MgSO₄ and evaporation gave 15.43 g of a dark oil. This was purified by chromatography on silica gel using a three component elutant which consisted of 80% CH₂Cl₂ and 20% of a gradient of EtOAc/hexane. This gave the product as a yellow solid: 3.32 g (34%); mp 68-72 °C, MS EI *m*/*e* 229, 231, 233 (M⁺);¹H NMR (400 MHz, DMSO-d₆) δ 3.99 (t, 2H, J=5.1 Hz), 4.34 (t, 2H, J=5.0 Hz), 6.51 (t, 1H, J=2.7 Hz), 6.53 (d, 1H, J=7.8 Hz), 7.04 (d, 1H, J=8.0 Hz), 7.29 (t, 1H, J=2.7 Hz), 11.43 (s, 1H).

| Elemental analysis for C₁₀H₉Cl₂NO | | | |
|---|---|---|---|
| Calc'd | C, 52.20; | H, 3.94; | N, 6.09. |
| Found | C, 52.09; | H, 3.92; | N, 5.96. |

### Intermediate 4

### 3,7-Dichloro-4-(2-chloroethoxy)-1H-indole

To a solution of 7-chloro-4-(2-chloroethoxy)-1H-indole (4.61 g, 20.0 mmol) in acetonitrile (100 mL) was added N-chlorosuccimide (2.94 g, 2.20 mmol) at room temperature. The reaction was allowed to stir for 1.5 hour then poured into water (100 mL) and extracted with methylene chloride (200 mL). The organic layer was dried over anhydrous magnesium sulfate, filtered, and the solvent removed under vacuum to afford a dark solid. This material was chromatographed (methylene chloride-hexanes: 1:2) to afford 4.15 g (78.4 %) as a white solid: mp 106-107.5 °C; IR (KBr) 3400 cm-1; MS EI *m*/*e* 263, 265, 267, 269 (M+); ¹H NMR (CDCl₃) δ 3.91 (2H, t, J=6.2 Hz), 4.33 (2H, t, J=6.2 Hz), 6.47 (1H, d, J= 8.4 Hz), 7.08-7.13 (2H, m), 8.26 (1H, bs, NH).

| Elemental analysis for C₂₂H₂₅N₂O₃Cl | | | |
|---|---|---|---|
| Calc'd | C, 65.91; | H, 6.28; | N, 6.99 |
| Found | C, 65.61; | H, 6.21; | N, 6.89 |

### EXAMPLE 1

### [2-(1H-Indol-4-yloxy)-ethyl]-(4-phenyl-butyl)-amine

A solution of the 2-(1H-indol-4-yloxy)-chloroethane (1.80 g, 9.20 mmol) and 4-phenyl-1-aminobutane (4.12g, 27.6 mmol) in anhydrous dimethylsulfoxide (25 mL) was heated to 80 °C for 6 hours. The reaction mixture was poured into water (150 mL) and extracted with methylene chloride (3x 100 mL). The organic layers were combined and dried over anhydrous magnesium sulfate, filtered, and the solvent concentrated. Purification by flash chromatography (5%-10% methanol-CH₂Cl₂) afforded 1.89 g (65.9%) of a tan oil: MS *m*/*e* 308 (M+). The oxalate salt was prepared in tetrahydrofuran: mp 202-204 °C.

| Elemental analysis for C₂₀H₂₄N₂O·C₂H₂O₄·0.5H₂O | | | |
|---|---|---|---|
| Calc'd | C, 64.85; | H, 6.68; | N, 6.87. |
| Found | C, 64.66; | H, 6.61; | N, 6.70. |

This general procedure utilizing 7-chloro-4-(2-chloroethoxy)-1H-indole or 3,7-dichloro-4-(2-chloroethoxy)-1H-indole and reacting with either benzylamine, 4-fluorobenzyl amine, 4-chlorobenzyl amine or thiophene-2-methylamine afforded:
(**1b**) Benzyl-[2-(7-chloro-1H-indol-4-yloxy)-ethyl]-amine (68%). The fumarate salt was prepared in isopropanol as colorless crystals; mp 168-170 °C; MS El *m*/*e* 300, 302 (M⁺).

| Elemental analysis for C₁₇H₁₇ClN₂O·0.5C₄H₄O₄·0.25C₃H₈O | | | |
|---|---|---|---|
| Calc'd | C, 63.45; | H, 5.66; | N, 7.49 |
| Found | C, 63.12; | H, 5.61; | N, 7.31. |

(**1c**) Benzyl-[2-(3,7-dichloro-1H-indol-4-yloxy)-ethyl]-amine (67.8 %): The fumarate salt was prepared and characterized: mp 201-202 °C; MS EI *m*/*e 334,* 336, 338 (M⁺).

| Elemental analysis for C₁₇H₁₆Cl₂N₂O·0.5C₄H₄O₄ | | | |
|---|---|---|---|
| Calc'd | C, 58.03; | H, 4.61; | N, 7.12. |
| Found | C, 57.88; | H, 4.45; | N, 6.96. |

(**1d**) 4-Fluorobenzyl-[2-(3,7-dichloro-1H-indol-4-yloxy)-ethyl]-amine (64.5 %): mp 102.5-103.5 °C.

| Elemental analysis for C₁₇H₁₅FCl₂N₂O | | | |
|---|---|---|---|
| Calc'd | C, 57.81; | H, 4.28; | N, 7.93. |
| Found | C, 57.68; | H, 4.16; | N, 7.86. |

(**1e**) 4-Chlorobenzyl-[2-(3,7-dichloro-1H-indol-4-yloxy)-ethyl]-amine (59.9 %): mp 115-116 °C; MS EI 368 *m*/*e* (M⁺).

| Elemental analysis for C₁₇H₁₅Cl₃N₂O·0.25H₂O | | | |
|---|---|---|---|
| Calc'd | C, 54.57; | H, 4.17 ; | N, 7.49. |
| Found | C, 54.43; | H, 3.82; | N, 7.32. |

(**1f**) Thien-2-ylmethyl-[2-(3,7-dichloro-1H-indol-4-yloxy)-ethyl]-amine (76.3%): mp 99-101 °C, MS EI 340, 342, 344 *m*/*e* (M+).

| Elemental analysis for C₁₅H₁₅Cl₂N₂OS | | | |
|---|---|---|---|
| Calc'd | C, 52.70; | H, 4.13 ; | N, 8.21. |
| Found | C, 52.70; | H, 3.95; | N, 8.19. |

### Intemediate 5

### N-Benzyl-N-[2-(1H-indol-3-(2,2,2-trifluoroethanoyl)-4-yloxy)-ethyl]-carbamic acid tert-butyl ester

To a stirring anhydrous solution of benzyl-[2-(1H-indol-4-yloxy)-ethyl]-carbamic acid tent-butyl ester (1.85 g, 5.05 mmol) and TEA (0.8 mL, 0.6 g, 6 mmol) in CH₂Cl₂ was added trifluoroacetic acid anhydride (1.1 mL, 1.6 g, 7.8 mmol) over 5 minutes at room temperature. The reaction mixture was stirred at room temperature over-night. It was washed twice with H₂O and then dried over MgS04. Evaporation of solvent gave 3.38g of residue. This was purified by chromatography on silica gel with a hexane/EtOAc gradient to give the title compound as an amorphous light yellow solid: 1.15g (49%); MS EI *m*/*e* 462 (M⁺); IR(KBr) 1719 cm⁻¹, 1744 cm⁻¹; ¹H NMR (400 MHz, DMSO-d6) δ 1.33 and 1.38 (2s, 9H, rotamers), 3.50-3.63 (2m, 2H, rotamers), 4.15 (t, 2H, J=5.5 Hz), 4.54 (s, 2H), 6.77 (d, 1H, J=7.9 Hz), 7.15 (d, 1H, J=8.1 Hz), 7.20-7.27 (m, 4H), 7.29-7.35 (m, 2H), 8.32 (s, 1H), 12.58 (s, 1H).

### Intermediate 6

### N-[2-(1H-indol-4-yloxy)-ethyl]-N-(4-phenyl-butyl)-trifluoroacetamide

To a solution of [2-(1H-indol-4-yloxy)-ethyl]-(4-phenyl-butyl)-amine (2.38 g, 7.72 mmol) and triethylamine (1.56 g, 15.4 mmol) in anhydrous methylene chloride (30 mL) at room temperature was slowly added trifluoroacetic anhydride (2.42 g, 11.6 mmol) over 10 minutes. The reaction was stirred for 1 hour and then poured into a 1:1 solution of saturated sodium carbonate-water (50 mL) and extracted with methylene chloride (2x100 mL). The organic layer dried over anhydrous magnesium sulfate, filtered, and the solvent evaporated. Purification by flash chromatography (20% ethyl acetate-hexanes) afforded 1.61 g (51.6%) of an off-white solid: mp 70-72 °C; MS *m*/*e* 404(M+); IR (KBr) 3360, 2950, 1725 cm⁻¹.

### Intermediates 7 & 8

### N-Benzyl-N-[2-(7-chloro-1H-indol-4-yloxy)-ethyl]-2,2,2-trifluoro-acetamide and N-Benzyl-N-[2-(7-chloro-3-trifluoroacetyl-1H-indol-4-yloxy)-ethyl]-2,2,2-trifluoro-acetamide

To a solution of benzyl-[2-(7-chloro-1H-indol-4-yloxy)-ethyl]-amine (4.55 g, 15.1 mmol) in CH₂Cl₂ (200 mL) at room temperature was added triethylamine (TEA) (2.15 mL, 1.56 g, 15.4 mmol) and then trifluoroacetic acid anhydride (4.5 mL, 6.7 g, 32 mmol) over 20 minutes. The solution was stirred at room temperature over-night. It was washed twice with H₂O. Drying over MgSO₄ and evaporation of the solvent gave 7.33 g of residue which consisted primarily of the two products. These were separated and purified by chromatography on silica gel with a gradient of CH₂Cl₂/hexane/EtOAc (10/80/10, 4/82/14, 0/86/14, 0/80/20) which first eluted N-benzyl-N-[2-(7-chloro-1H-indol-4-yloxy)-ethyl]-2,2,2-trifluoro-acetamide as light yellow crystals: 2.79 g (47%); mp 114-116 °C; MS El *m*/*e* 396 (M⁺); IR (KBr) 1682 cm⁻¹; ¹H NMR (400 MHz, CDCl₃) δ 3.79 and 3.86 (2t, 2H, J=5.6 Hz, J=5.0 Hz, rotamers), 4.28 and 4.31 (2t, 2H, J=5.6 Hz, J=5.0 Hz, rotamers), 4.89 and 4.93 (2s, 2H, rotamers), 6.38 and 6.40 (2d, 1H, J=8.3 Hz, J=8.5 Hz, rotamers), 6.64-6.68 (m, 1H), 7.05 and 7.08 (2d, 1H, J=8.1 Hz, J=8.3 Hz, rotamers); 7.19-7.44 (m, 6H), 8,42 (s, 1H).

| Elemental analysis for C₁₉H₁₆ClF₃N₂O₂ | | | |
|---|---|---|---|
| Calc'd | C, 57.51; | H, 4.06; | N, 7.06. |
| Found | C, 57.11; | H, 3.88; | N, 7.01. |

N-Benzyl-N-[2-(7-chloro-3-trifluoroacetyl-1H-indol-4-yloxy)-ethyl]-2,2,2-trifluoro-acetamide was then eluted off the column to afford 3.18 g (43%) of crystalline solid; mp 152-154 °C; MS FAB *m*/*e* 493 (MH⁺); IR (KBr) 1685 cm⁻¹, 1699 cm⁻¹; ¹H NMR (400 MHz, CDCl₃) δ 3.84 and 4.01 (2t, 2H, J=5.0 Hz, J=5.3 Hz, rotamers), 4.26 and 4.31 (2t, 2H, J=5.5 Hz, J=5.0 Hz, rotamers), 4.92 and 5.00 (2s, 2H, rotamers), 6.63 and 6.66 (2d, 1H, J=8.8 Hz, J=8.8 Hz, rotamers), 7.27-7.42 (m, 6H), 8.04-8.08 (m, 1H), 9.13 (s, 1H).

| Elemental analysis for C₂₁H₁₅ClF₆N₂O₃ | | | |
|---|---|---|---|
| Calcd | C, 51.18; | H, 3.07; | N, 5.68. |
| Found | C, 51.31; | H, 2.89; | N, 5.58. |

### Intermediate 9

### N-Benzyl-N-[2-(3-chloro-1H-indol-4-yloxy)-ethyl]-carbamic acid tert-butyl ester

To a solution of N-benzyl-N-[2-(1H-indol-4-yloxy)-ethyl]-carbamic acid tert-butyl ester (6.3 g, 17.2 mmol) in tetrahydrofuran (100 mL) was added N-chlorosuccinimide (2.3 g, 17.2 mmol) in two portions over 1 hour. The reaction was allowed to stir for 18 hours and the solvent removed under vacuum. The mixture was dissolved in diethyl ether and the insoluble solids filtered. The solvent was again removed and the product purified by chromatography (30% ethyl acetate-hexanes) to afford 5.65 g of white solid (81.9 %): mp 114-116 °C.

| Elemental analysis for C₂₂H₂₅N₂O₃Cl | | | |
|---|---|---|---|
| Calc'd | C, 65.91; | H, 6.28; | N, 6.99 |
| Found | C, 65.61; | H, 6.21; | N, 6.89 |

This general procedure utilizing N-methyl-N-[2-(1H-indol-4-yloxy)-ethyl]-carbamic acid tent-butyl ester, N-[2-(1H-Indol-4-yloxy)-ethyl]-phthalimide, N-benzyl-N-[2-(7-chloro-1H-indol-4-yloxy)-ethyl]-2,2,2-trifluoro-acetamide and [2-(1H-indol-4-yloxy)-ethyl]-(4-phenyl-butyl)-trifluoroacetamide afforded, respectively:
(**9b**) N-Methyl-N-[2-(3-chloro-1H-indol-4-yloxy)-ethyl]-carbamic acid ten-butyl ester as a white solid: (74.9 %); mp 153-154 °C; MS FAB *m*/*z 325* (M⁺+H⁺).

| Elemental analysis for C₁₆H₂₁N₂O₃Cl | | | |
|---|---|---|---|
| Calc'd | C, 59.17; | H, 6.52; | N, 8.62 |
| Found | C, 59.08; | H, 6.33; | N, 8.49 |

(**9c**) N-[2-(3-Chloro-1H-indol-4-yloxy)-ethyl]-phthalimide as yellowish white crystals: mp 161-163 °C.

| Elemental analysis for C₁₈H₁₃N₂O₃·0.33H₂O | | | |
|---|---|---|---|
| Calc'd | C, 62.36; | H, 3.97; | N, 8.08 |
| Found | C, 62.37; | H, 3.68; | N, 8.07 |

(**9d**) N-Benzyl-N-[2-(3,7-dichloro-1H-indol-4-yloxy)-ethyl]-2,2,2-trifluoro-acetamide as a white solid: (82%); mp 156-158 °C; MS EI *m*/*e* 430, 432, 434 (M⁺); IR(KBr) 1680 cm^{-1; 1}H NMR (400 MHz, CDCl₃) δ 3.76 and 3.81 (2t, 2H, J=1.3 Hz, J=1.4 Hz, rotamers), 4.14 and 4.15 (2t, 2H, J=1.5 Hz, J=1.6 Hz, rotamers), 4.95 and 4.96 (2s, 2H, rotamers), 6.41 and 6.43 (2d, 1H, J=8.4 Hz, J=8.7 Hz, rotamers), 7.095 and 7.097 (2d, 1H, J=8.2 Hz, J=8.2 Hz, rotamers), 7.16 (d, 1H, J=2.5 Hz), 7.22-7.41 (m, 5H), 8.27-8.35 (m, 1H).

| Elemental analysis for C₁₉H₁₅Cl₂F₃N₂O₂ | | | |
|---|---|---|---|
| Calc'd | C, 52.92; | H, 3.51; | N, 6.50 |
| Found | C, 52.54; | H, 3.26; | N, 6.29. |

(**9e**) N-[2-(3-chloro-1H-indol-4-yloxy)-ethyl]-N-(4-phenyl-butyl)-trifluoroacetamide: (71.4 %), mp 113-114°C; MS *m*/*e* 438 (M+).

| Elemental analysis for C₂₂H₂₂N₂O₂ClF₃ | | | |
|---|---|---|---|
| Calc'd | C, 60.21; | H, 5.05; | N, 6.38 |
| Found | C, 60.51; | H, 4.94; | N, 6.31. |

### EXAMPLE 2

### [2-(3-Chloro-1H-indol-4-yloxy)-ethyl]-(4-phenyl-butyl)-amine

A mixture of [2-(3-chloro-1H-indol-4-yloxy)-ethyl]-(4-phenyl-butyl)-trifluoro-acetamide(1.15 g, 2.62 mmol) and potassium carbonate (2.53 g, mmol) in a solution of methanol-water ( 50 mL:3 mL) was heated to reflux for 3 hours. The solvent was removed under vacuum and the crude product was dissolved in methylene chloride (150 mL) and washed with water (100 mL). The aqueous layer was extracted again with methylene chloride (100 mL) and the combined organic layers dried over anhydrous magnesium sulfate, filtered, and the solvent evaporated. The product was purified by flash chromatography (5% methanol-methylene chloride) to afford 847 mg (94.3 %) of a tan oil: MS *m*/*e* 342 (M+), 344 (M+). The fumarate salt was prepared in isopropanol: mp 195-196 °C.

| Elemental analysis for C₂₀H₂₃N₂OCl·0.5C₄H₄O₄ | | | |
|---|---|---|---|
| Calc'd | C, 65.91; | H, 6.29; | N, 6.99 |
| Found | C, 66.15; | H, 6.38; | N, 6.81. |

This general procedure utilizing N-benzyl-N-[2-(3,7-dichloro-1H-indol-4-yloxy)-ethyl]-2,2,2-trifluoro-acetamide, and N-benzyl-N-[2-(7-chloro-1H-indol-4-yl-oxy)-ethyl]-2,2,2-trifluoro-acetamide afforded, respectively:
(**2b**) Benzyl-[2-(3,7-dichloro-1H-indol-4-yloxy)-ethyl]-amine, 92 %. The fumarate salt was prepared from ethanol as a white powder, mp 201-202 °C; MS EI *m*/*e* 334, 336, 338 (M⁺).

| Elemental analysis for C₁₇H₁₆Cl₂N₂O·0.5C₄H₄O₄ | | | |
|---|---|---|---|
| Calc'd | C, 58.03; | H, 4.61; | N, 7.12. |
| Found | C, 57.88; | H, 4.45; | N, 6.96. |

(**2c**) 1-[4-(2-Benzylamino-ethoxy)-7-chloro-1H-indol-3-yl]-2,2,2-trifluoro-ethanone: (80 %). The fumarate salt was prepared in ethanol: mp 215 °C (dec); MS FAB *m*/*e* 397 (MH⁺).

| Elemental analysis for C₁₉H₁₆ClF₃N₂O₂·0.5C₄H₄O₄ | | | |
|---|---|---|---|
| Calc'd | C, 55.46; | H, 3.99; | N, 6.16. |
| Found | C, 55.24; | H, 3.80; | N, 6.08. |

### EXAMPLE 3

### 1-[4-(2-Benzylamino-ethoxy)-1H-indol-3-yl]-2,2,2-trifluoro-ethanone

To a solution of N-benzyl-N-[2-(1H-indol-3-(2,2,2-trifluoroethanoyl)-4-yl-oxy)-ethyl]-carbamic acid tert-butyl ester (1.1 g, 2.4 mmol) in methylene chloride (60mL) was added trifluoroacetic acid (TFA) (0.21 g, 1.8 mmol). Thin layer chromatography (TLC) (CH₂Cl₂/CH₃OH, 88/12v/v) showed no change after 1 hour at room temperature. TFA (0.74 g, 6.5 mmol) was added and the mixture stirred 2 hours. Some product was then visible in the TLC. TFA (0.86 g, 7.5 mmol) was added and the mixture was stirred overnight. TLC showed some starting material. TFA (0.06 g, 0.5 mmol) was added and the mixture was stirred for 1 hour. The reaction mixture was washed once with saturated NaHCO₃ (30-40 mL). It was dried over MgSO₄. Evaporation of the solvent gave 1.02 g of residue. This was purified by chromatography on silica gel with a gradient of CH₂Cl₂/CH₃OH (96/4 and 95/5) to give the product as a light tan oil (0.78 g, 90%).

To a hot solution of fumaric acid (0.2557 g, 2.203 mmol) in EtOH (15 mL) was added a hot solution of the base in EtOH (15 mL). This mixture stood at room temperature for 2 hours. It was filtered to give the title compound as a white powder: 0.5398 g (54%); decomp. >220 °C; MS EI *mle* 362 (M⁺); IR (KBr) 1660 cm⁻¹.

| Elemental analysis for C₁₉H₁₇F₃N₂O₂·0.5C₄H₄O₄ | | | |
|---|---|---|---|
| Calc'd | C, 60.00; | H, 4.56; | N, 6.66. |
| Found | C, 60.12; | H, 4.40; | N, 6.75. |

This general procedure utilizing benzyl-[2-(1H-indol-4-yloxy)-ethyl]-carbamic acid ten-butyl ester afforded
**(3b)** N-Benzyl-[2-(1H-indol-4-yloxy)]-ethylamine, (26 %). The fumarate salt was prepared in isopropanol: mp 158-165 °C.

| Elemental analysis for C₁₇H₁₈N₂O·C₄H₄O₄ | | | |
|---|---|---|---|
| Calc'd | C, 65.96; | H, 5.80; | N, 7.33. |
| Found | C, 65.94; | H, 5.87; | N, 7.19. |

Affinity for the dopamine autoreceptor was established by a modification of the standard experimental test procedure of Seemen and Schaus, European Journal of Pharmacology 203, 105-109 (1991), wherein homogenized rat striatal brain tissue is incubated with ³H-quinpirole (Quin.)and various concentrations of test compound, filtered and washed and counted in a Betaplate scintillation counter.

High affinity for the dopamine D-2 receptor was established by the standard experimental test procedure of Fields, et al., Brain Res., 136, 578 (1977) and Yamamura et al., eds., Neurotransmitter Receptor Binding, Raven Press, N.Y. (1978) wherein homogenized limbic brain tissue is incubated with ³H-spiroperidol (Spiper.) and various concentrations of test compound, filtered and washed and shaken with Hydrofluor scintillation cocktail (National Diagnostics) and counted in a Packard 460 CD scintillation counter.

High affinity for the serotonin 5-HT_{1A} receptor was established by testing the claimed compound's ability to displace [³H] 8-OHDPAT (dipropylaminotetralin) from the 5-HT_{1A} serotonin receptor following the procedure of Hall et al., J. Neurochem. 44, 1685 (1985). This procedure is employed to analogize this property of the claimed compounds with that of buspirone, which is a standard for anxiolytic activity, and, like the compounds of this invention, displays potent affinity for the 5-HT_{1A} serotonin receptor subtype. The anxiolytic activity of buspirone is believed to be, at least partially, due to its 5-HT_{1A} receptor affinity (Vander Maclen et al., Eur. J. Pharmacol. 1986, 129 (1-2) 133-130).

The results of these standard experimental test procedures were as follows:

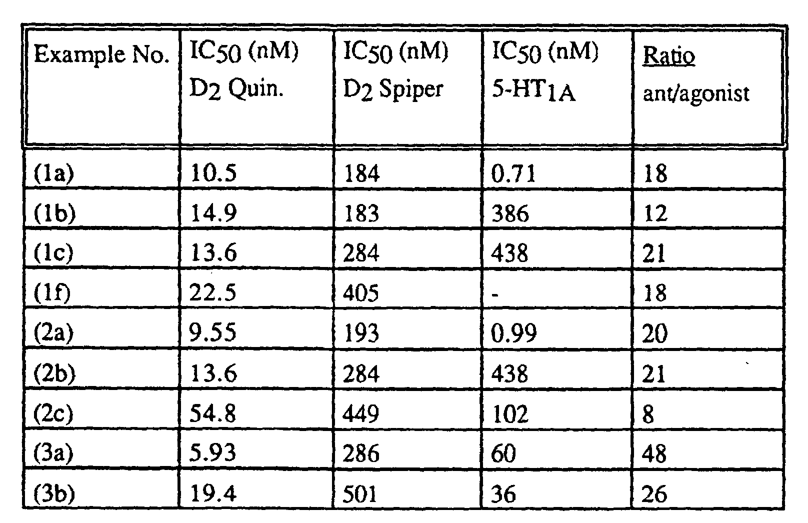

Hence, the compounds of this invention effect the synthesis of the neurotransmitter dopamine and thus are useful in the treatment of dopaminergic disorders such as schizophrenia, Parkinson's disease, Tourette's Syndrome, alcohol addiction, cocaine addiction, and addiction to analagous drugs. These compounds also have affinity for the 5-HT_{1A} receptors and therefore have the ability to modulate serotinergic activity. As such, they are also useful in the treatment of diseases characterized by disturbances in the serotinergic systems, such as anxiety, stress, depression, sexual dysfunctions and sleep disturbances.

Applicable solid carriers for pharmaceutical compositions containing the compounds of this invention can include one or more substances which may also act as flavoring agents, lubricants, solubilizers, suspending agents, fillers, glidants, compression aids, binders or tablet-disintergrating agents or an encapsulating material. In powders, the carrier is a finely divided solid which is in admixture with the finely divided active ingredient. In tablets, the active ingredient is mixed with a carrier having the necessary compression properties in suitable proportions and compacted in the shape and size desired. The powders and tablets preferably contain up to 99% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Liquid carriers may be used in preparing solutions, suspensions, emulsions, syrups and elixirs. The active ingredient of this invention can be dissolved or suspended in a pharmaceutically acceptable liquid carrier such as water, an organic solvent, a mixture of both or pharmaceutically acceptable oils or fat. The liquid carrier can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example, intramuscular, intraperitoneal or subcutaneous injection. Sterile solutions can also be administered intravenously. Oral administration may be in either liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form.

The dosage to be used in the treatment of a specific psychosis must be subjectively determined by the attending physician. The variables involved include the specific psychosis and the size, age and response pattern of the patient.

## Claims

1. A compound of formula I 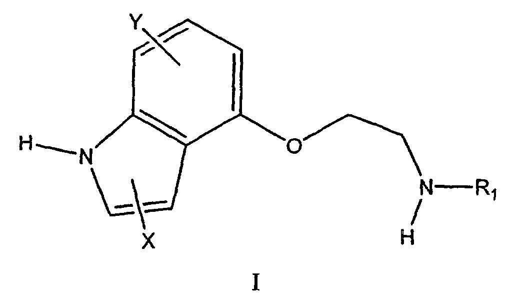 in which:
R₁ is hydrogen, alkyl of 1 to 10 carbon atoms, cycloalkylalkyl of 6 to 12 carbon atoms, arylalkyl of 7 to 12 carbon atoms, (haloaryl)alkyl of 7 to 12 carbon atoms, (alkoxyaryl)alkyl of 8 to 12 carbon atoms, thienylmethyl, furanylmethyl, pyridinylmethyl, alkylphenyl of 7 to 12 carbon atoms, 4-fluorobutyrophenone or 6-fluoro-1,2-benzisoxazol-yl-propyl;
X is hydrogen, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms; or a pharmaceutically acceptable salt thereof; provided that when R₁ and X are both hydrogen, then Y is not hydrogen, 5-methoxy or 7-methoxy.

2. A compound of Claim 1 in which
R₁ is hydrogen, alkyl of 1 to 10 carbon atoms, cyclohexylmethyl, arylalkyl of 7 to 12 carbon atoms, (haloaryl)alkyl of 7 to 12 carbon atoms or (alkoxyaryl)alkyl of 8 to 12 carbon atoms;
X is H, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms; or a pharmaceutically acceptable salt thereof.

3. A compound of Claim 1 in which R₁ is alkyl of 1 to 6 carbon atoms, benzyl, halobenzyl, alkoxybenzyl of 8 to 12 carbon atoms or alkylbenzyl of 8 to 12 carbon atoms; X is hydrogen, halogen or trifluoroacetyl and Y is hydrogen or halogen; or a pharmaceutically acceptable salt thereof.

4. The compound of Claim 1 which is [2-(1H-indol-4-yloxy)-ethyl]-(4-phenyl-butyl)-amine or a pharmaceutically acceptable salt thereof.

5. The compound of Claim 1 which is benzyl-[2-(7-chloro-1H-indol-4-yloxy)-ethyl]-amine or a pharmaceutically acceptable salt thereof.

6. The compound of Claim 1 which is benzyl-[2-(3,7-dichloro-1H-indol-4-yloxy)-ethyl]-amine or a pharmaceutically acceptable salt thereof.

7. The compound of Claim 1 which is 4-fluorobenzyl-[2-(3,7-dichloro-1H-indol-4-yloxy)-ethyl]-amine or a pharmaceutically acceptable salt thereof.

8. The compound of Claim 1 which is 4-chlorobenzyl-[2-(3,7-dichloro-1H-indol-4-yloxy)-ethyl]-amine or a pharmaceutically acceptable salt thereof.

9. The compound of Claim 1 which is thien-2-ylmethyl-[2-(3,7-dichloro-1H-indol-4-yloxy)-ethyl]-amine or a pharmaceutically acceptable salt thereof.

10. The compound of Claim 1 which is [2-(3-chloro-1H-indol-4-yloxy)-ethyl]-(4-phenyl-butyl)-amine or a pharmaceutically acceptable salt thereof.

11. The compound of Claim 1 which is benzyl-[2-(3,7-dichloro-1H-indol-4-yloxy)-ethyl]-amine or a pharmaceutically acceptable salt thereof.

12. The compound of Claim 1 which is 1-[4-(2-benzylamino-ethoxy)-7-chloro-1H-indol-3-yl]-2,2,2-trifluoro-ethanone or a pharmaceutically acceptable salt thereof.

13. The compound of Claim 1 which is 1-[4-(2-benzylamino-ethoxy)-1H-indol-3-yl]-2,2,2-trifluoro-ethanone or a pharmaceutically acceptable salt thereof.

14. The compound of Claim 1 which is (N-benzyl-[2-(1H-indol-4-yloxy)]-ethyl-amine or a pharmaceutically acceptable salt thereof.

15. A pharmaceutical composition of matter comprising a compound of the formula: 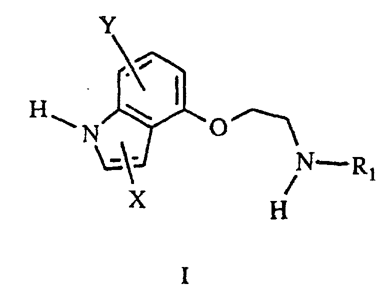 in which:
R₁ is hydrogen, alkyl of 1 to 10 carbon atoms, cycloalkylalkyl of 6 to 12 carbon atoms, arylalkyl of 7 to 12 carbon atoms, (haloaryl)alkyl of 7 to 12 carbon atoms, (alkoxyaryl)alkyl of 8 to 12 carbon atoms, thienylmethyl, furanylmethyl, pyridinylmethyl, alkylphenyl of 7 to 12 carbon atoms, 4-fluorobutyrophenone or 6-fluoro-1,2-benzisoxazol-yl-propyl;
X is hydrogen, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms;
or a pharmaceutically acceptable salt thereof and a pharmaceutically acceptable carrier therefor.

16. A compound of the formula I: 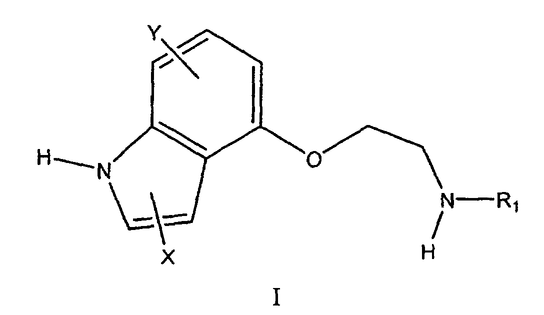 in which :
R₁ is hydrogen, alkyl of 1 to 10 carbon atoms, cycloalkylalkyl of 6 to 12 carbon atoms, arylalkyl of 7 to 12 carbon atoms, (haloaryl)alkyl of 7 to 12 carbon atoms, (alkoxyaryl)alkyl of 8 to 12 carbon atoms, thienylmethyl, furanylmethyl, pyridinylmethyl, alkylphenyl of 7 to 12 carbon atoms, 4-fluorobutyrophenone or 6-fluoro-1,2-benzisoxazol-yl-propyl;
X is hydrogen, halogen, cyano, alkyl of 1 to 6 carbon atoms, acetyl, trifluoroacetyl, trifluoromethyl or formyl;
Y is hydrogen, halogen, alkoxy of 1 to 6 carbon atoms or alkyl of 1 to 6 carbon atoms; or a pharmaceutically acceptable salt thereof; for use in therapy.

17. A compound according to claim 16 in which the therapy is treatment of diseases **characterized by** disturbances in the dopaminergic and serotinergic systems.

18. A compound according to claim 16 in which the therapy is selected from: schizophrenia, reduction of dopamine synthesis and release in a patient suffering from hyperactivity of the dopaminergic systems, Parkinson's disease, Tourette's Syndrome, alcohol addition, cocaine addition, anxiety, stress, depression, sexual dysfunctions and sleep disturbances.

19. Use of a compound of formula I as defined in claim 16 for the preparation of a medicament for treatment of diseases **characterized by** disturbances in the dopaminergic and serotinergic systems.

20. Use of a compound of formula I as defined in claim 16 for the preparation of a medicament for treatment of schizophrenia, reduction of dopamine synthesis and release in a patient suffering from hyperactivity of the dopaminergic systems, Parkinson's disease, Tourette's Syndrome, alcohol addition, cocaine addition, anxiety, stress, depression, sexual dysfunctions or sleep disturbances

## Patentansprüche

1. Verbindung der Formel I 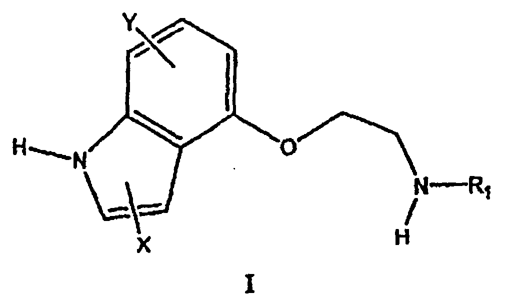 worin:
R₁ für Wasserstoff, Alkyl mit 1 bis 10 Kohlenstoffatomen, Cycloalkylalkyl mit 6 bis 12 Kohlenstoffatomen, Arylalkyl mit 7 bis 12 Kohlenstoffatomen, (Halogenaryl)alkyl mit 7 bis 12 Kohlenstoffatomen, (Alkoxyaryl)alkyl mit 8 bis 12 Kohlenstoffatomen, Thienylmethyl, Furanylmethyl, Pyridinylmethyl, Alkylphenyl mit 7 bis 12 Kohlenstoffatomen, 4-Fluorbutyrophenon oder 6-Fluor-1,2-benzisoxazol-yl-propyl steht;
X Wasserstoff, Halogen, Cyano, Alkyl mit 1 bis 6 Kohlenstoffatomen, Acetyl, Trifluoracetyl, Trifluormethyl oder Formyl darstellt;
Y Wasserstoff, Halogen, Alkoxy mit 1 bis 6 Kohlenstoffatomen oder Alkyl mit 1 bis 6 Kohlenstoffatomen darstellt; oder ein pharmazeutisch annehmbares Salz davon; unter der Bedingung, dass wenn R₁ und X beide Wasserstoff darstellen, Y nicht Wasserstoff, 5-Methoxy oder 7-Methoxy darstellt.

2. Verbindung nach Anspruch 1, worin:
R₁ für Wasserstoff, Alkyl mit 1 bis 10 Kohlenstoffatomen, Cyclohexylmethyl, Arylalkyl mit 7 bis 12 Kohlenstoffatomen, (Halogenaryl)alkyl mit 7 bis 12 Kohlenstoffatomen oder (Alkoxyaryl)alkyl mit 8 bis 12 Kohlenstoffatomen steht;
X für H, Halogen, Cyano, Alkyl mit 1 bis 6 Kohlenstoffatomen, Acetyl, Trifluoracetyl, Trifluormethyl oder Formyl steht;
Y Wasserstoff, Halogen, Alkoxy mit 1 bis 6 Kohlenstoffatomen oder Alkyl mit 1 bis 6 Kohlenstoffatomen darstellt; oder ein pharmazeutisch annehmbares Salz davon.

3. Verbindung nach Anspruch 1, worin R₁ Alkyl mit 1 bis 6 Kohlenstoffatomen, Benzyl, Halogenbenzyl, Alkoxybenzyl mit 8 bis 12 Kohlenstoffatomen oder Alkylbenzyl mit 8 bis 12 Kohlenstoffatomen darstellt; X für Wasserstoff, Halogen oder Trifluoracetyl steht und Y Wasserstoff oder Halogen darstellt; oder ein pharmazeutisch annehmbares Salz davon.

4. Verbindung nach Anspruch 1, welche [2-(1H-Indol-4-yloxy)-ethyl]-(4-phenyl-butyl)-amin oder ein pharmazeutisch annehmbares Salz davon ist.

5. Verbindung nach Anspruch 1, welche Benzyl-[2-(7-chlor-1Hindol-4-yloxy)-ethyl]-amin oder ein pharmazeutisch annehmbares Salz davon ist.

6. Verbindung nach Anspruch 1, welche Benzyl-[2-(3,7-dichlor-1H-indol-4-yloxy)-ethyl]-amin oder ein pharmazeutisch annehmbares Salz davon ist.

7. Verbindung nach Anspruch 1, welche 4-Fluorbenzyl-[2-(3,7-dichlor-1H-indol-4-yloxy)-ethyl]-amin oder ein pharmazeutisch annehmbares Salz davon ist.

8. Verbindung nach Anspruch 1, welche 4-Chlorbenzyl-[2-(3,7-dichlor-1H-indol-4-yloxy)-ethyl]-amin oder ein pharmazeutisch annehmbares Salz davon ist.

9. Verbindung nach Anspruch 1, welche Thien-2-ylmethyl-[2-(3,7-dichlor-1H-indol-4-yloxy)-ethyl]-amino oder ein pharmazeutisch annehmbares Salz davon ist.

10. Verbindung nach Anspruch 1, welche [2-(3-Chlor-1H-indol-4-yloxy)-ethyl]-(4-phenyl-butyl)-amin oder ein pharmazeutisch annehmbares Salz davon ist.

11. Verbindung nach Anspruch 1, welche Benzyl-[2-(3,7-dichlor-1H-indol-4-yloxy)-ethyl]-amin oder ein pharmazeutisch annehmbares Salz davon ist.

12. Verbindung nach Anspruch 1, welche 1-[4-(2-Benzylaminoethoxy)-7-chlor-1H-indol-3-yl]-2,2,2-trifluor-ethanon oder ein pharmazeutisch annehmbares Salz davon ist.

13. Verbindung nach Anspruch 1, welche 1-[4-(2-Benzylaminoethoxy)-1H-indol-3-yl]-2,2,2-trifluor-ethanon oder ein pharmazeutisch annehmbares Salz davon ist.

14. Verbindung nach Anspruch 1, welche (N-Benzyl-[2-(1H-indol-4-yloxy)]-ethyl-amin oder ein pharmazeutisch annehmbares Salz davon ist.

15. Pharmazeutische Zusammensetzung aus Substanzen, welche eine Verbindung der Formel: 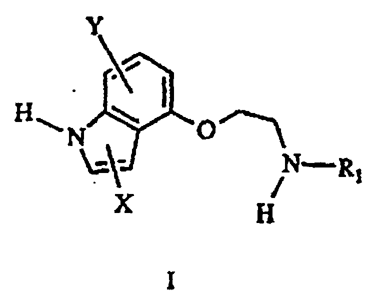 worin:
R₁ für Wasserstoff, Alkyl mit 1 bis 10 Kohlenstoffatomen, Cycloalkylalkyl mit 6 bis 12 Kohlenstoffatomen, Arylalkyl mit 7 bis 12 Kohlenstoffatomen, (Halogenaryl)alkyl mit 7 bis 12 Kohlenstoffatomen, (Alkoxyaryl)alkyl mit 8 bis 12 Kohlenstoffatomen, Thienylmethyl, Furanylmethyl, Pyridinylmethyl, Alkylphenyl mit 7 bis 12 Kohlenstoffatomen, 4-Fluorbutyrophenon oder 6-Fluor-1,2-benzisoxazol-yl-propyl steht;
X Wasserstoff, Halogen, Cyano, Alkyl mit 1 bis 6 Kohlenstoffatomen, Acetyl, Trifluoracetyl, Trifluormethyl oder Formyl darstellt;
Y Wasserstoff, Halogen, Alkoxy mit 1 bis 6 Kohlenstoffatomen oder Alkyl mit 1 bis 6 Kohlenstoffatomen darstellt;
oder ein pharmazeutisch annehmbares Salz davon und einen pharmazeutisch annehmbaren Träger dafür umfasst.

16. Verbindung der Formel I: 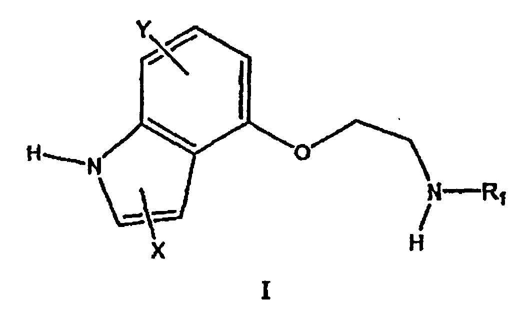 worin:
R₁ für Wasserstoff, Alkyl mit 1 bis 10 Kohlenstoffatomen, Cycloalkylalkyl mit 6 bis 12 Kohlenstoffatomen, Arylalkyl mit 7 bis 12 Kohlenstoffatomen, (Halogenaryl)alkyl mit 7 bis 12 Kohlenstoffatomen, (Alkoxyaryl)alkyl mit 8 bis 12 Kohlenstoffatomen, Thienylmethyl, Furanylmethyl, Pyridinylmethyl, Alkylphenyl mit 7 bis 12 Kohlenstoffatomen, 4-Fluorbutyrophenon oder 6-Fluor-1,2-benzisoxazol-yl-propyl steht;
X Wasserstoff, Halogen, Cyano, Alkyl mit 1 bis 6 Kohlenstoffatomen, Acetyl, Trifluoracetyl, Trifluormethyl oder Formyl darstellt;
Y Wasserstoff, Halogen, Alkoxy mit 1 bis 6 Kohlenstoffatomen oder Alkyl mit 1 bis 6 Kohlenstoffatomen darstellt;
oder ein pharmazeutisch annehmbares Salz davon; zur Verwendung in der Therapie.

17. Verbindung gemäß Anspruch 16, wobei die Therapie Behandlung von Krankheiten ist, welche durch Störungen in den dopaminergen und serotinergen Systemen gekennzeichnet sind.

18. Verbindung gemäß Anspruch 16, wobei die Therapie ausgewählt wird aus: Schizophrenie, Reduktion der Dopamin-Synthese und Abgabe in einem Patienten, welcher an Hyperaktivität des dopaminergen Systems leidet, Parkinson-Erkrankung, Tourette-Syndrom, Alkoholabhängigkeit, Kokainabhängigkeit, Angstzuständen, Stress, Depression, sexuellen Fehlfunktionen und Schlafstörungen.

19. Verwendung einer Verbindung der Formel I, wie in Anspruch 16 definiert, für die Herstellung eines Medikaments zur Behandlung von Krankheiten, welche durch Störungen in den dopaminergen und serotinergen Systemen gekennzeichnet sind.

20. Verwendung einer Verbindung der Formel I wie in Anspruch 16 definiert für die Herstellung eines Medikaments zur Behandlung von Schizophrenie, Reduktion der Dopamin-Synthese und Abgabe in einem Patienten, welcher an Hyperaktivität des dopaminergen Systems leidet, Parkinson-Erkrankung, Tourette-Syndrom, Alkoholabhängigkeit, Kokainabhängigkeit, Angstzuständen, Stress, Depression, sexuellen Fehlfunktionen oder Schlafstörungen.

## Revendications

1. Composé de formule I 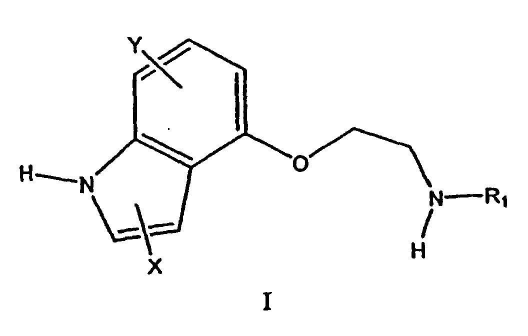 dans laquelle :
R₁ est un hydrogène, un alkyle de 1 à 10 atomes de carbone, un cycloalkylalkyle de 6 à 12 atomes de carbone, un arylalkyle de 7 à 12 atomes de carbone, un (halogénoaryl)alkyle de 7 à 12 atomes de carbone, un (alcoxyaryl)alkyle de 8 à 12 atomes de carbone, un thiénylméthyle, un furanylméthyle, un pyridinylméthyle, un alkylphényle de 7 à 12 atomes de carbone, une 4-fluorobutyrophénone ou un 6-fluoro-1,2-benzisoxazolyl-propyle;
X est un hydrogène, un halogène, un cyano, un alkyle de 1 à 6 atomes de carbone, un acétyle, un trifluoroacétyle, un trifluorométhyle ou un formyle;
Y est un hydrogène, un halogène, un alcoxy de 1 à 6 atomes de carbone ou un alkyle de 1 à 6 atomes de carbone; ou un sel pharmaceutiquement acceptable de celui-ci; à condition que lorsque R₁ et X sont tous deux un hydrogène, alors Y n'est pas un hydrogène, un 5-méthoxy ou un 7-méthoxy.

2. Composé suivant la revendication 1 dans lequel
R₁ est un hydrogène, un alkyle de 1 à 10 atomes de carbone, un cyclohexylméthyle, un arylalkyle de 7 à 12 atomes de carbone, un (halogénoaryl)alkyle de 7 à 12 atomes de carbone ou un (alcoxyaryl)alkyle de 8 à 12 atomes de carbone;
X est H, un halogène, un cyano, un alkyle de 1 à 6 atomes de carbone, un acétyle, un trifluoroacétyle, un trifluorométhyle ou un formyle;
Y est un hydrogène, un halogène, un alcoxy de 1 à 6 atomes de carbone ou un alkyle de 1 à 6 atomes de carbone; ou un sel pharmaceutiquement acceptable de celui-ci.

3. Composé suivant la revendication 1, dans lequel R₁ est un alkyle de 1 à 6 atomes de carbone, un benzyle, un halogénobenzyle, un alcoxybenzyle de 8 à 12 atomes de carbone, ou un alkylbenzyle de 8 à 12 atomes de carbone; X est un hydrogène, un halogène ou un trifluoroacétyle et Y est un hydrogène ou un halogène; ou un sel pharmaceutiquement acceptable de celui-ci.

4. Composé suivant la revendication 1, qui est la [2-(1H-indol-4-yloxy)éthyl]-(4-phénylbutyl)amine ou un sel pharmaceutiquement acceptable de celle-ci.

5. Composé suivant la revendication 1, qui est la benzyl-[2-(7-chloro-1H-indol-4-yloxy)éthyl]amine ou un sel pharmaceutiquement acceptable de celle-ci.

6. Composé suivant la revendication 1, qui est la benzyl-[2-(3,7-dichloro-1H-indol-4-yloxy)éthyl]-amine ou un sel pharmaceutiquement acceptable de celle-ci.

7. Composé suivant la revendication 1, qui est la 4-fluorobenzyl-[2-(3,7-dichloro-1H-indol-4-yloxy)éthyl]aminé ou un sel pharmaceutiquement acceptable de celle-ci.

8. Composé suivant la revendication 1, qui est la 4-chlorobenzyl-[2-(3,7-dichloro-1H-indol-4-yloxy)éthyl]amine ou un sel pharmaceutiquement acceptable de celle-ci.

9. Composé suivant la revendication 1, qui est la thién-2-ylméthyl-[2-(3,7-dichloro-1H-indol-4-yloxy)éthyl]amine ou un sel pharmaceutiquement acceptable de celle-ci.

10. Composé suivant la revendication 1, qui est la [2-(3-chloro-1H-indol-4-yloxy)éthyl]-(4-phényl-butyl)amine ou un sel pharmaceutiquement acceptable de celle-ci.

11. Composé suivant la revendication 1, qui est la benzyl-[2-(3,7-dichloro-1H-indol-4-yloxy)éthyl]amine ou un sel pharmaceutiquement acceptable de celle-ci.

12. Composé suivant la revendication 1, qui est la 1-[4-(2-benzylaminoéthoxy)-7-chloro-1H-indol-3-yl]-2,2,2-trifluoroéthanone ou un sel pharmaceutiquement acceptable de celle-ci.

13. Composé suivant la revendication 1, qui est la 1-[4-(2-benzylaminoéthoxy)-1H-indol-3-yl]-2,2,2-trifluoroéthanone ou un sel pharmaceutiquement acceptable de celle-ci.

14. Composé suivant la revendication 1, qui est la (N-benzyl-[2-(1H-indol-4-yloxy)]éthylamine ou un sel pharmaceutiquement acceptable de celle-ci.

15. Composition pharmaceutique de matière comprenant un composé de formule : 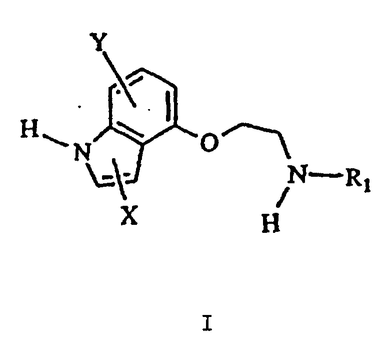 dans laquelle
R₁ est un hydrogène, un alkyle de 1 à 10 atomes de carbone, un cycloalkylalkyle de 6 à 12 atomes de carbone, un arylalkyle de 7 à 12 atomes de carbone, un (halogénoaryl)alkyle de 7 à 12 atomes de carbone, un (alcoxyaryl)alkyle de 8 à 12 atomes de carbone, un thiénylméthyle, un furanylméthyle, un pyridinylméthyle, un alkylphényle de 7 à 12 atomes de carbone, une 4-fluorobutyrophénone ou un 6-fluoro-1,2-benzisoxazolylpropyle;
X est un hydrogène, un halogène, un cyano, un alkyle de 1 à 6 atomes de carbone, un acétyle, un trifluoroacétyle, un trifluorométhyle ou un formyle;
Y est un hydrogène, un halogène, un alcoxy de 1 à 6 atomes de carbone ou un alkyle de 1 à 6 atomes de carbone;
ou un sel pharmaceutiquement acceptable de celui-ci et un excipient pharmaceutiquement acceptable pour celui-ci.

16. Composé de formule I : 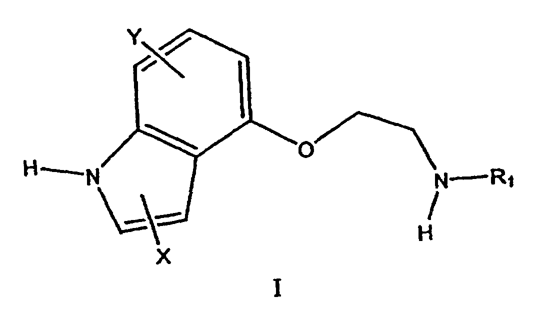 dans laquelle :
R₁ est un hydrogène, un alkyle de 1 à 10 atomes de carbone, un cycloalkylalkyle de 6 à 12 atomes de carbone, un arylalkyle de 7 à 12 atomes de carbone, un (halogénoaryl)alkyle de 7 à 12 atomes de carbone, un (alcoxyaryl)alkyle de 8 à 12 atomes de carbone, un thiénylméthyle, un furanylméthyle, un pyridinylméthyle, un alkylphényle de 7 à 12 atomes de carbone, une 4-fluorobutyrophénone ou un 6-fluoro-1,2-benzisoxazolylpropyle;
X est un hydrogène, un halogène, un cyano, un alkyle de 1 à 6 atomes de carbone, un acétyle, un trifluoroacétyle, un trifluorométhyle ou un formyle;
Y est un hydrogène, un halogène, un alcoxy de 1 à 6 atomes de carbone ou un alkyle de 1 à 6 atomes de carbone; ou un sel pharmaceutiquement acceptable de celui-ci; pour une utilisation en thérapie.

17. Composé suivant la revendication 16, dans lequel la thérapie est un traitement de maladies **caractérisées par** des perturbations des systèmes dopaminergiques et sérotoninergiques.

18. Composé suivant la revendication 16, dans lequel la thérapie est sélectionnée parmi : une schizophrénie, une réduction de la synthèse et de la libération de dopamine chez un patient souffrant d'une hyperactivité des systèmes dopaminergiques, de la maladie de Parkinson, du syndrome de Tourette, de l'alcoolisme, d'une dépendance à la cocaïne, de l'anxiété, du stress, de la dépression, de dysfonctionnements sexuels et des perturbations du sommeil.

19. Utilisation d'un composé de formule I comme défini à la revendication 16, pour la préparation d'un médicament pour le traitement de maladies **caractérisées par** des perturbations dans les systèmes dopaminergiques et sérotoninergiques.

20. Utilisation d'un composé de formule I, comme défini à la revendication 16, pour la préparation d'un médicament pour le traitement d'une schizophrénie, d'une réduction de la synthèse et de la libération de dopamine chez un patient souffrant d'une hyperactivité des systèmes dopaminergiques, de la maladie de Parkinson, du syndrome de Tourette, de l'alcoolisme, d'une dépendance à la cocaïne, de l'anxiété, du stress, de la dépression, de dysfonctionnements sexuels et de perturbations du sommeil.
